# EUROPEAN PATENT APPLICATION

(11) **EP 1 847 241 A2**
(43) Date of publication of application: **24.10.2007**
(21) Application number: 07014650.1
(22) Date of filing: 17.01.1997
(51) Int. Cl.: A61F 13/02

(54) **Dressing mechanism**

(30) Priority: 10.05.1996 US 17258 P
(62) Divisional of application: 03021906.7
(71) Applicant: Beaudry, Wallace J., Elkhart Lake, WI 53020 (US)
(72) Inventor: Beaudry, Wallace J., Elkhart Lake, WI 53020 (US)
(74) Representative: Sparing, Rolf Klaus

(57) **Abstract**

The invention relates to a dressing mechanism in accordance to the preamble of claim 1, comprising a first section (120; 301), a second section (110; 325) and a third section (120; 303), the first section (120; 301) being coupled to the second section (110; 325) and the second section (110) being coupled to the third section (120; 303); wherein the first section (301) and the third section (303) each have a first side, a predetermined portion of the first side having an adhesive coating, the second section (110; 321) comprising an elastic material including a plurality of openings (64; 312; 316). A dressing mechanism which permits a safe and persistent holding on an epidermal area is achieved in that the first section (120; 301) and the third section (120; 303) are each laminated materials comprising at least a first layer (319) and a second layer and in that the first layer (319) includes the first side.

## Description

The invention relates to a dressing mechanism in accordance to the preamble of claim 1.

FR 928, 389 describes an dressing mechanism for the treatment of wounds, in particular to wounds which need to be contracted by an outer force, comprising a first and a second section each including an adhesive material and a third section coupled to the first section and the second section wherein the third section comprises an elastic material having three openings

US 5,116,675 describes an dressing mechanism for the treatment of wounds comprising a first section and a second section designed as adhesive strips, wherein a third section is coupled to the first section and the second section. The third section comprises an elastic material.

FR 1.203.376 describes an elastic dressing mechanism for the treatment of wounds, comprising a central bandage of rectangular shape which has adhesive stripes connected to ist border regions in order to fixate the bandage to the human skin.

US 3,085,024 describes a dressing mechanism for the treatment of wounds, comprising a bandage structure which is fastened by a first elastic material and a second elastic material each designed as a tape and each containing a plurality of perforations forming tear zones.

It is the object of the invention to provide a dressing mechanism, which permits a safe and persisting holding on an epidermal area.

The invention achieves this object for the dressing mechanism mentioned at the beginning by the characterizing features of claim 1.

With respect to the invention's applications as a dressing the invention may be generally described as comprising: a first section, a second section, and a third section. Of these three sections, the first section is coupled to the second section and the second section is coupled to the third section. The second section comprising an elastic material with the first section and the third section each having a first side ; and a predetermined portion of the first side including an adhesive located thereon. The second section of the invention may include a plurality of openings of a predetermined size and predetermined shape.

It should be noted that the predetermined shape or shapes of the openings may be spatially organized in a predetermined manner respective to each other. This is because in one embodiment of the present invention the second section is located between the first and third sections and is preferably composed of an elastic material. By placing openings in the elastic material at predetermined locations the strength of the elastic material, when the elastic material is stretched, may be varied and the distribution of force across the elastic material may by varied. Also, the openings can be used to provide a visual reference to a user of the amount of stress being placed upon the second section and whether or not that section has been stretched sufficiently or been stretched too much since the shape of the openings will change in response to the degree to which the elastic material is stretched. Such a visual reference would be useful to medical personnel where, e.g., it is desirable for a predetermined amount of pressure to be applied to a wound.

Further, the second section includes a first margin (if the second section is round then there is structurally just one annular margin near at least a portion of the perimeter of the second section) a second margin. The first section may be integral or coupled to the second section at the first margin; and the third section may be integral or coupled to the second section at the second margin.

Preferably, but not necessarily, the first section and the third section are laminated materials comprising a first layer, a second intermediate layer, and a third layer : with the third layer including the first side coated with adhesive and protected prior to use by a silicone release liner. The second section includes a first margin and a second margin. The first section includes a first channel located between the first layer and the third layer of the first section for receiving the first margin. The second section includes a second channel located between the first layer and the third layer of the second section for receiving the second margin. The second intermediate layer comprising an adhesive material. The first margin and the second margin of the second section respectively including at least one opening and the first margin engaging the second intermediate layer in the first channel and the adhesive material extending through the opening of the first margin; and the second margin engaging the second intermediate layer in the second channel and the adhesive material extending through the opening of the second margin.

The first and third layer of the first section and the first and third layer of the third section preferably being an inelastic material in some embodiments. The inelastic material may be of any suitable material such as a TYVEC brand type of material.

Alternatively, the dressing mechanism may be described as comprising: a first section, a second section, and a third section such that the first section is coupled to the second section and the second section is coupled to the third section. The first section and the third section comprising an elastic material and the first section and the third section each having a first side ; and a predetermined portion of the first side including an adhesive located thereon.

Further, the second section includes at least one opening of a predetermined size and the first section and the third section each include at least one opening comprising a predetermined shape. As previously noted the openings of predetermined shape are spatially organized in a predetermined manner respective to each other.

Also, the second section may include at least one margin and the first section and the third section each have a respective margin area. The first section margin is coupled to the second section at a first predetermined portion the margin of the second section. The third section margin being coupled to the second section at a second predetermined portion of the margin of the second section.

Preferably, the second section is a laminated material comprising at least a first layer, a second intermediate layer, and a third layer; the third layer including the first side. The first section and the third section including a first section margin and a third section margin. Both the first section margin and the third section margin being composed of an elastic material. The second section including at least one channel located between the first layer and the third layer of the second section at the second section margin for receiving the margins of the first and third sections. The second intermediate layer comprising an adhesive material. The first section margin and the third section margin respectively including at least one opening and the margins of the first and third sections engaging the second intermediate layer in the channel at the respective first predetermined margin area and second predetermined margin area so that the adhesive material extends through the openings formed in the material which makes up the first and third section margins. The first and third layer of the second section may, in this embodiment, comprises an inelastic material. The inelastic material may be a polyester.

Further, the second section includes at least one opening or at least one generally transparent section to either allow the wound or burn to be exposed to the air to be observed visually. Additionally, the second section could be modified to include a mechanism for irrigating the wound or burn under the bandage so that the wound or burn could be cleaned or treated without having to remove the dressing. Also, at least one side of the second section could be designed so that it is capable of isolating the wound in a clean environment by creating a solid antiseptic barrier around the wound through the use of a colloid type adhesive or be capable of contacting a wound or burn so that medicine could be applied to the wound or burn directly.

Further, the present invention may be described as a method for using a dressing mechanism where the dressing mechanism comprises a first section, a second section, and a third section; the first section being coupled to the second section and the second section being coupled to the third section; the first section and the third section comprising an elastic material; the first section and the third section each having a first side; and a predetermined portion of the first side including an adhesive located thereon. The method consequently comprising: First, applying the first section to a first predetermined location on an epidermis. Second, pulling the third section toward a second predetermined location on the epidermis. Third, applying the third section to the second predetermined location on the epidermis.
Figure 19 is a top plan view of an alternative embodiment of the present invention including an elastic strip.
Figure 20 is a top plan view of an alternative embodiment of the present invention showing an embodiment having application for only one side of a persons nose or for raising a predetermined portion of an epidermal layer.
Figure 21 is a top plan view of an alternative embodiment of the present invention showing the elastic member having a first end coupled to a pod and a second end coupled to an anchor mechanism for application to a selected area of a person or animal epidermis. For example, the anchor mechanism could be applied to a persons cheek and the pod applied to the epidermis of a persons nose to enhance opening of the nasal passage.
Figure 22 is a side elevational view illustrating embodiment of Figure 21 with the anchor mechanism applied to a persons cheek and the pod applied to a side of a persons nose.
Figure 23 top plan view which illustrates the embodiment of Figure 21 in use to hold an incision open.
Figure 24 top plan view which illustrates the embodiment of Figure 19 in use to keep an incision closed.
Figure 25 top plan view which illustrates the embodiment Figure 19 in use to keep an incision closed with the ends of the incision kept in proper alignment to add in suturing the incision.
Figure 26 is a side elevational view showing the embodiment of either Figure 21 or Figure 19 being used on persons nose as a nasal dilator to enhance breathing. The embodiment of Figure 19 is believed to be preferable to the embodiment of Figure 21 for this purpose although either could be used.
Figure 27 is a perspective view generally showing a human nose.
Figure 28 is a cross sectional view of the nose in Figure 27 with the nose shown absent any nasal dilator.
Figure 29 is a cross sectional view of the nose in Figure 27 with the nose shown being in a state of relatively little air flow through the nasal passages.
Figure 30 is a cross sectional view of the nose m Figure 27 with a nasal dilator of the present invention applied illustrating an appreciable air flow through the nasal passages.
Figure 34 is a top plan view of an alternative structure to the embodiment illustrated in Figure 19.
Figure 35 is a perspective view of an another alternative structure of the present invention.
Figure 36 is a top plan view of the embodiment disclosed in Figure 35.
Figure 37 is a perspective view of an another alternative structure of the present invention.
Figure 38 is a top plan view of the embodiment disclosed in Figure 37.
Figure 39 is a perspective view of an another alternative structure of the present invention.
Figure 40 is a top plan view of the embodiment disclosed in Figure 39.
Figure 41 is a top plan view another embodiment of the present invention illustrating an embodiment of the present invention by supper-imposing two views of the embodiment; the phantom lines showing the embodiment at rest without the latex sections being stretched and the solid lines illustrating the latex sections being stretched while the center or second section maintains position over the treatment area despite the uneven tension applied to the various anchor sections.
Figure 42 is a top plan view of the embodiment shown in Figure 41 illustrating how the second center section may be positioned and various anchoring sections positioned to adjust the stress or pressure applied at the center section.
Figure 43 is a perspective view of another embodiment of the present invention.
Figure 44 is a top plan view of the embodiment disclosed in Figure 43.
Figure 45 is a perspective view of an another alternative structure of the present invention.
Figure 46 is a top plan view of the embodiment disclosed in Figure 45.
Figure 47 is a top plan view illustrating how force may be distributed in two directions in a particular embodiment of the present invention.
Figure 48 is a top plan view illustrating how force may be distributed in four directions in a particular embodiment of the present invention.
Figure 49 is a perspective view of an another alternative structure of the present invention.
Figure 50 is a top plan view of the structure disclosed in Figure 49.
Figure 51 is a perspective view of an another alternative structure of the present invention.
Figure 52 is a top plan view of the structure disclosed in Figure 51.
Figure 53 is a perspective view of an another alternative structure of the present invention.
Figure 54 is a top plan view of the an alternative embodiment to the structure disclosed in Figure 53.
Figure 55 is a perspective view of an another alternative structure of the present invention.
Figure 56 is a top plan view of an alternative embodiment of the alternative structure shown in Figure 55.
Figure 57 is a top plan view showing the structure disclosed in Figure 55 applied over the incision of a wound and acting as a guide for suturing the wound.
Figure 58 is a top plan view showing the two of the structures disclosed in Figure 55 being used to hold a wound open.
Figure 59 is a perspective view showing the structure disclosed in Figure 55 being used for guiding an intravenous tube and holding the tube in a predetermined position.
Figure 60 is a top plan view showing the structure disclosed in Figure 55 holding the edges of a wound or incision together.
Figure 61 is a perspective view of an another alternative structure of the present invention.
Figure 62 is a top plan view of the structure disclosed in Figure 61.
Figure 63 is a perspective view of an another alternative structure of the present invention.
Figure 64 is a top plan view of the structure disclosed in Figure 63.
Figure 65 is a perspective view of an another alternative structure of the present invention.
Figure 66 is a top plan view of the structure disclosed in Figure 65.
Figure 67 is a perspective view of an another alternative structure of the present invention.
Figure 68 is a view from line 68-68 of Figure 69.
Figure 69 is a top plan view of the structure disclosed in Figure 67.
Figure 70 is a perspective view of an another alternative structure of the present invention.
Figure 71 is a top plan view of the structure disclosed in Figure 70.
Figure 72 is a perspective view of an another alternative structure of the present invention.
Figure 73 is a top plan view of the structure disclosed in Figure 74.
Figure 74 is a top plan view of another alternative embodiment of the present invention.
Figure 75 is a perspective view of an another alternative structure of the present invention.
Figure 76 is a top plan view of the structure disclosed in Figure 75.
Figure 77 is a cross-sectional view from line 77--77 of Figure 84.
Figure 78 is a cross-sectional view from line 78-78 of Figure 86.
Figure 79 is a cross-sectional view from line 79-79 of Figure 86.
Figure 80 is a cross-sectional view of a structure similar to the structure disclosed in Figure 86 illustrating the use of input and output ports which may be used to irrigate a wound or deliver medicine to a predetermined area.
Figure 81 is a view taken from line 81-81 of Figure 39.
Figure 82 is a view taken from line 82-82 of Figure 40
Figure 83 is a top plan view of another alternative embodiment of the present invention.
Figure 84 is a perspective view of the alternative structure of the present invention disclosed in Figure 83.
Figure 85 is a top plan view of another alternative embodiment of the present invention.
Figure 86 is a perspective view of the alternative structure of the present invention disclosed in Figure 85.
Figure 87 is a top plan view of another alternative embodiment of the present invention.
Figure 88 is a top plan view of another alternative embodiment of the present invention.
Figure 89 is an illustration showing how the embodiment disclosed in Figure 70 may be used on an area of the human body that is subject to a high degree of movement.
Figure 90 is an illustration showing how another alternative embodiment of the present invention may be used on an area of the human body that is subject to a high degree of movement.
Figure 91 illustrates how another alternative embodiment of the present invention may be used as a nasal dilator.
Figure 92 illustrates another method by which the alternative embodiment of the present invention shown in Figure 91 may be used as a nasal dilator.
Figure 93 illustrates how the embodiment shown in Figure 91 may be used to hold a flap of skin, in this case a human ear flap, in a predetermined position. This is useful where its is desired to have easy access to an area that might otherwise be blocked by a fold or flap of skin thus making work on that area difficult or cumbersome.

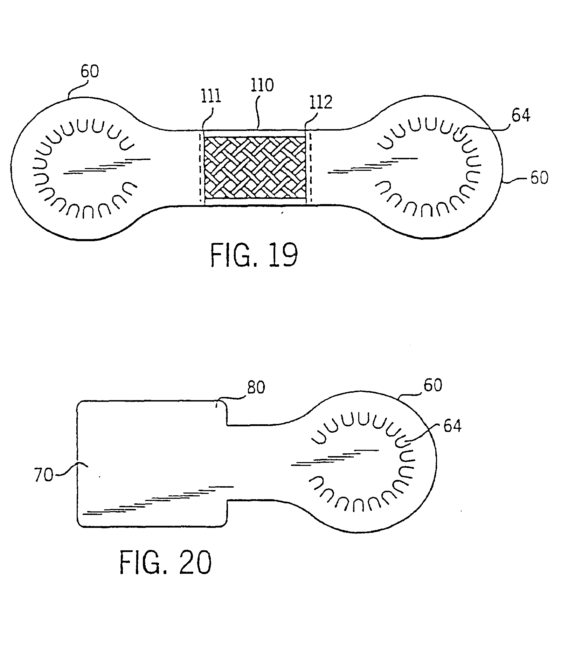

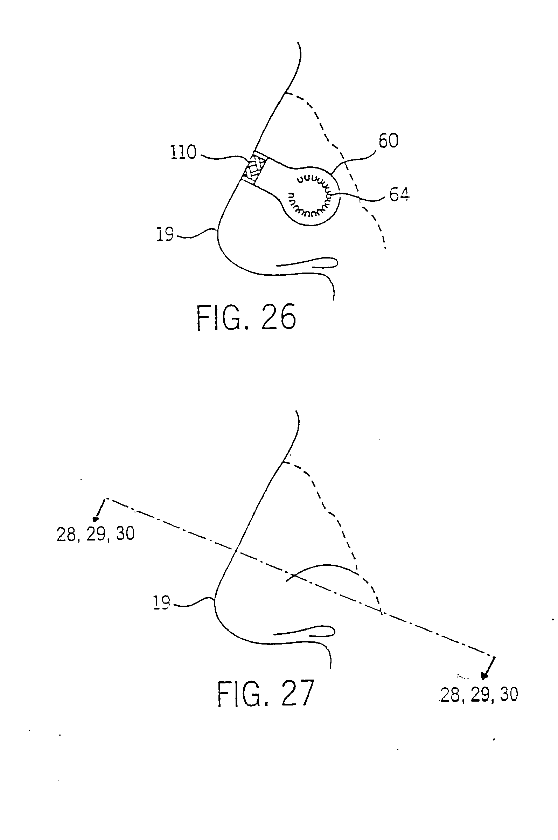

Referring now to Figure 19, a top plan view of an alternative embodiment of the present invention 10 may be seen to comprise an elastic midsection 110 having ends 111 and 112. Ends 111 and 112 are coupled to pod sections 60. This embodiment does not include any spring mechanism other than the elastic section 110; The elastic section 110 taking the place of the spring mechanism. The resiliency of the elastic section 110 will cause the two nasal pods 60 to be drawn together when the elastic member contracts. If this is done over a fulcrum point such as the bridge of the nose it will cause a lifting of the nasal passages and thus may be used as a nasal dilator as illustrated in Figure 26.

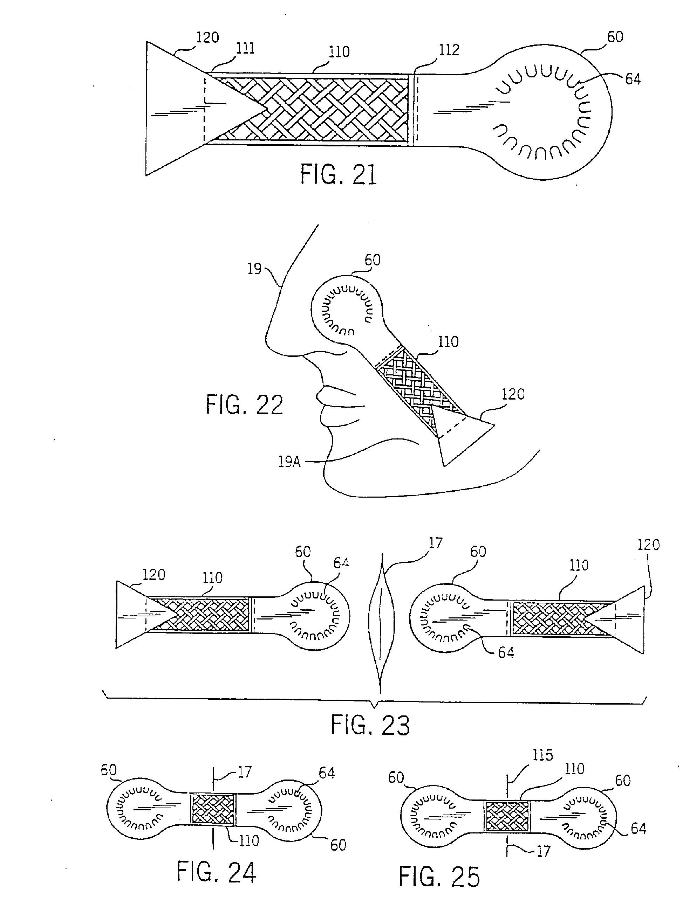

Additionally the mechanism of Figure 19 may be used as shown in Figures 24 and 25 to aid in holding a wound or incision 17 closed either for the purposes of healing as illustrated in Figure 24 or for the purpose of aiding in suturing as illustrated in Figure 25. The pods 60 adhering to the epidermis to either side of the wound and the elastic member 110 being stretched across the wound so that it will contract and draw the two pods 60 towards each other thereby closing the wound in an effective manner. Additionally, when the wound is closed in this manner a surgeon or physician may have both hands free to apply sutures 115 along the wound or incision 17. This is believed particularly helpful when dealing with a large wound or incision.

With respect to the embodiment of the invention shown in Figure 19 it should be noted that U shaped cuts or incisions 64 are also illustrated. Again, these cuts or incisions may be of any shape although the U or horseshoe shape is preferred however the embodiment disclosed in Figure 19 could function without these U shaped cuts or incisions 64.

Referring now to Figure 20 an alternative embodiment of the present invention for use as a nasal dilator is shown. In this embodiment the spring sections are included as shown in Figure 18 although they are not shown in Figure 20. This embodiment functions in a manner similar to the embodiment Figure 10 and is simply meant to illustrate once again that the nasal dilator of the present invention could be applied to only one side of a persons nose 19.

Referring now to Figure 21 another alternative embodiment of the present invention is shown in a top plan view illustrating the elastic member 110 coupled at its end 112 to pod 60 and coupled at its end 111 to an anchor 120. The anchor 120 has an adhesive layer applied to it in the same manner as the adhesive layer which is applied to the pod 60. The embodiment of the invention 10 shown in Figure 21 has application for maintaining an incision opening or wound opening for either a surgical procedure or cleansing purposes as illustrated in Figure 23 or for use as a nasal dilator for application to only side of a persons nose as illustrated in Figure 22.

Referring to Figure 22 pod 60 may be seen applied to the side of a persons nose 19 and elastic member 110 is stretched so that anchor 120 may be applied to the side of persons face 19A. Thus, elastic member 110 will contract and pull pod 60 and anchor 120 toward one another but since anchor 120 is positioned on a substantially stationary epidermal area of the persons face the majority of the movement will occur at pod 60 causing the epidermal area to which it is applied to be pulled outward and thus open the nasal passage.

Referring to Figure 23, the incision 17 may be seen to be held open by the action of the embodiment disclosed in Figure 21. The anchors 120 are applied to a substantially stationary epidermal area and the elastic members 110 are stretched and the pods 60 are positioned to either side of the wound or incision to hold it open so that the wound may be cleansed or a surgical procedure may be performed through the incision thus freeing the physician's hands for this purpose.

It should be noted that the U shaped cuts 64 are disclosed in the embodiment of the present invention 10 shown in Figure 21. While these U shaped cuts are preferred they are not considered necessary to practice the present invention.

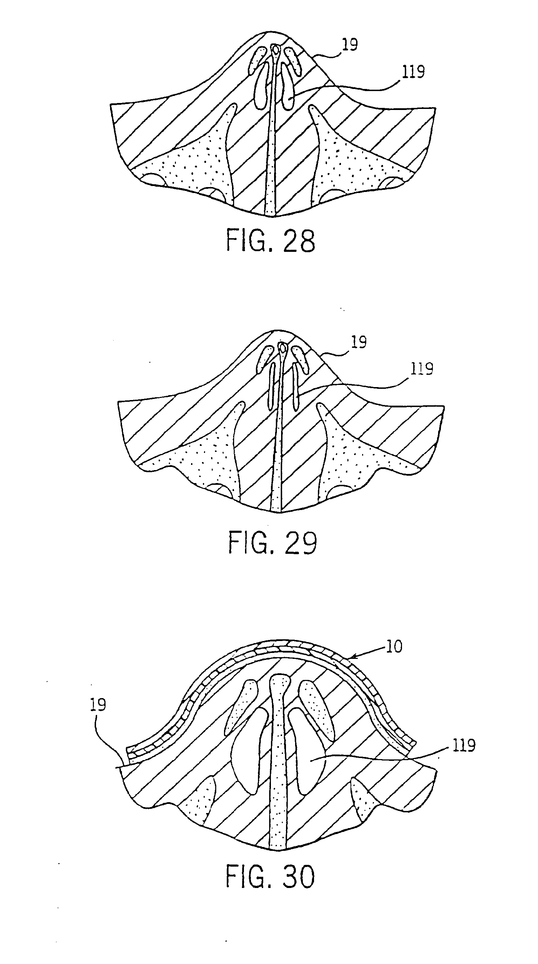

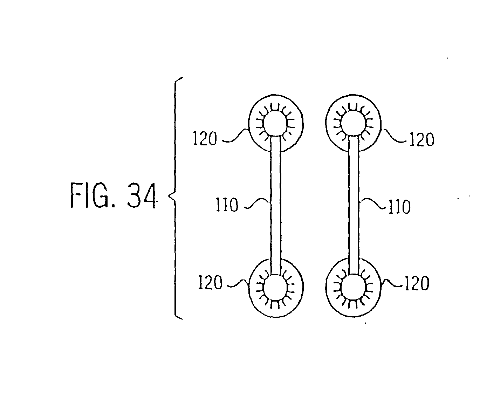

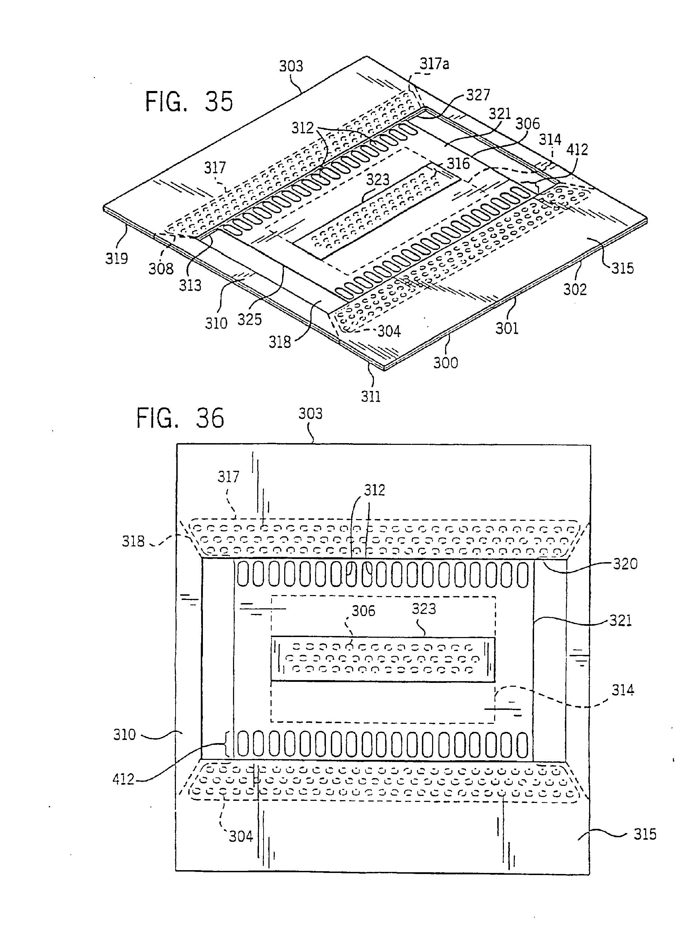

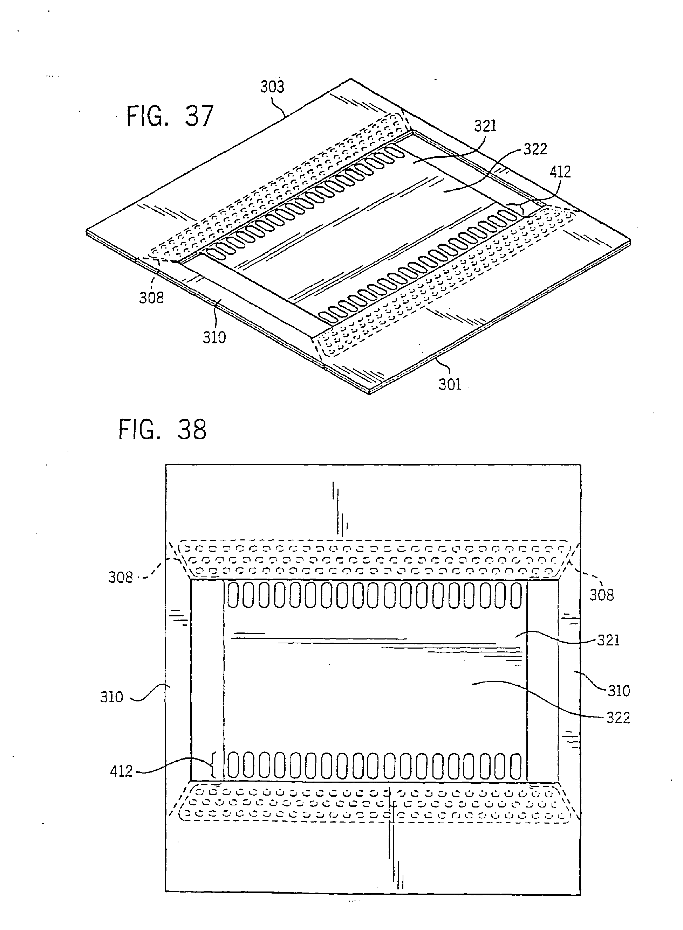

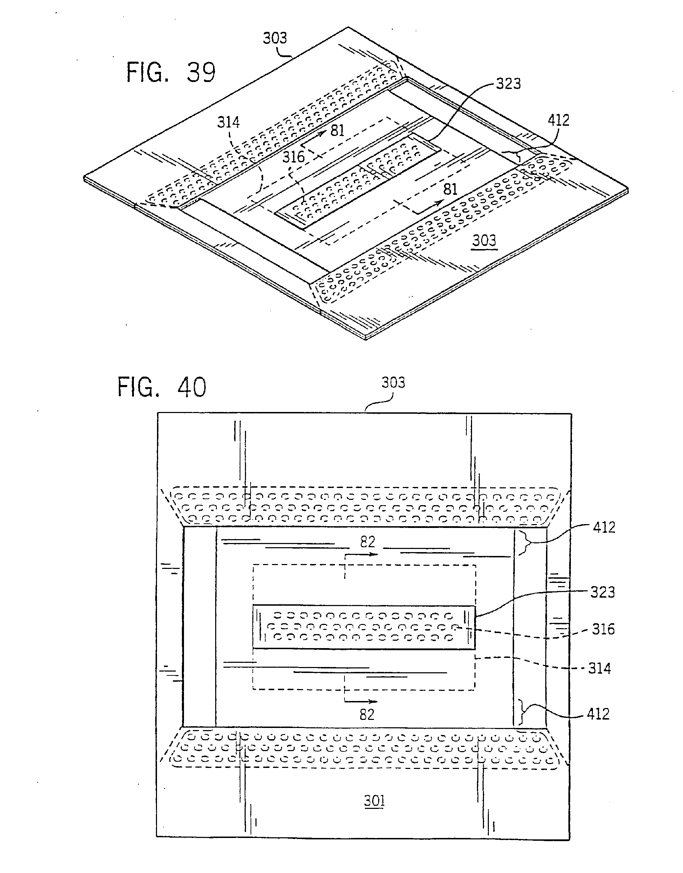

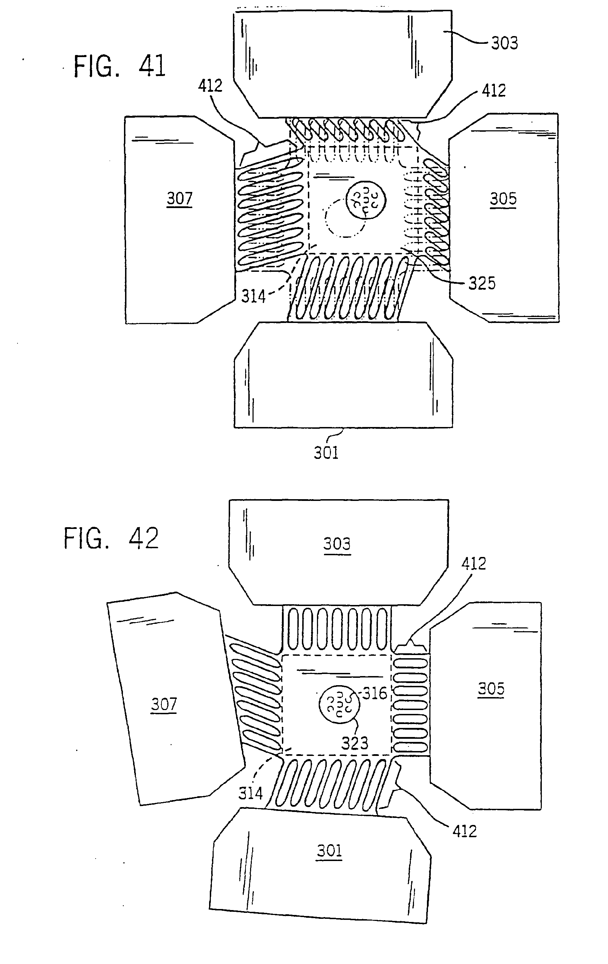

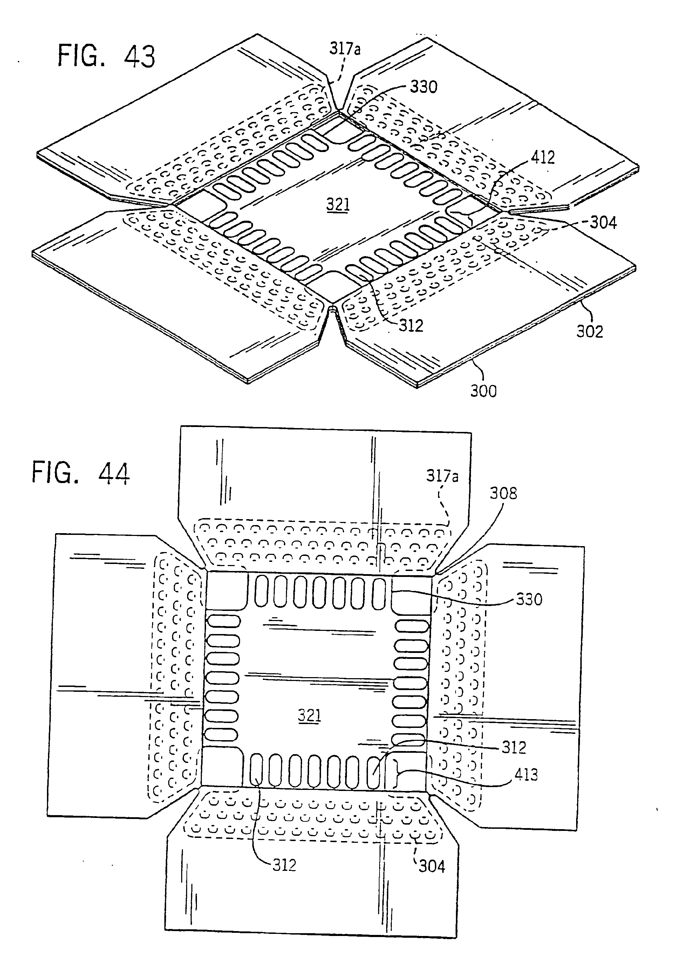

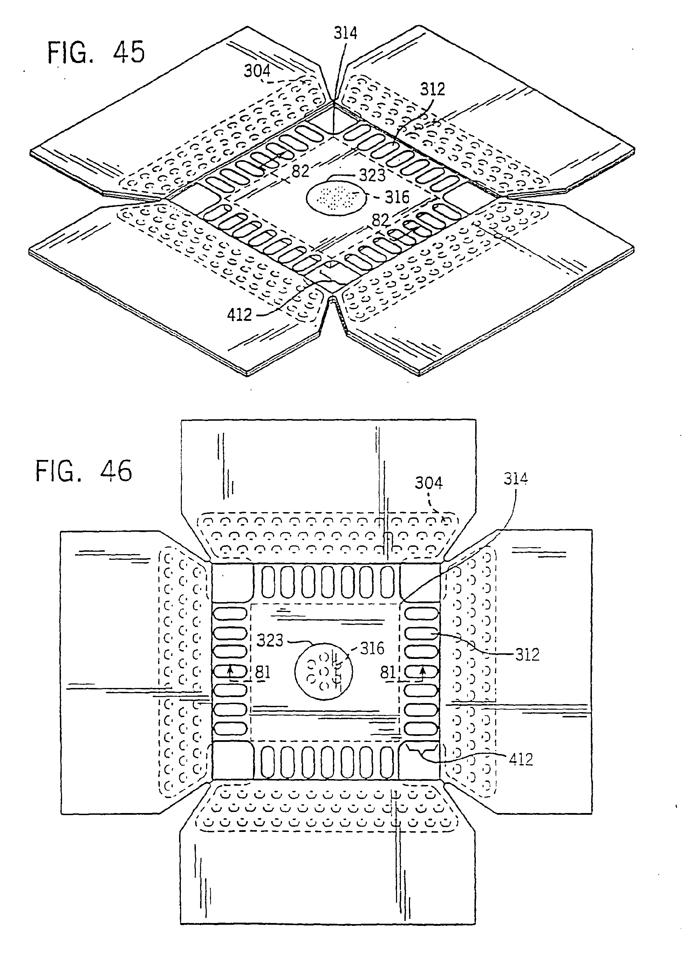

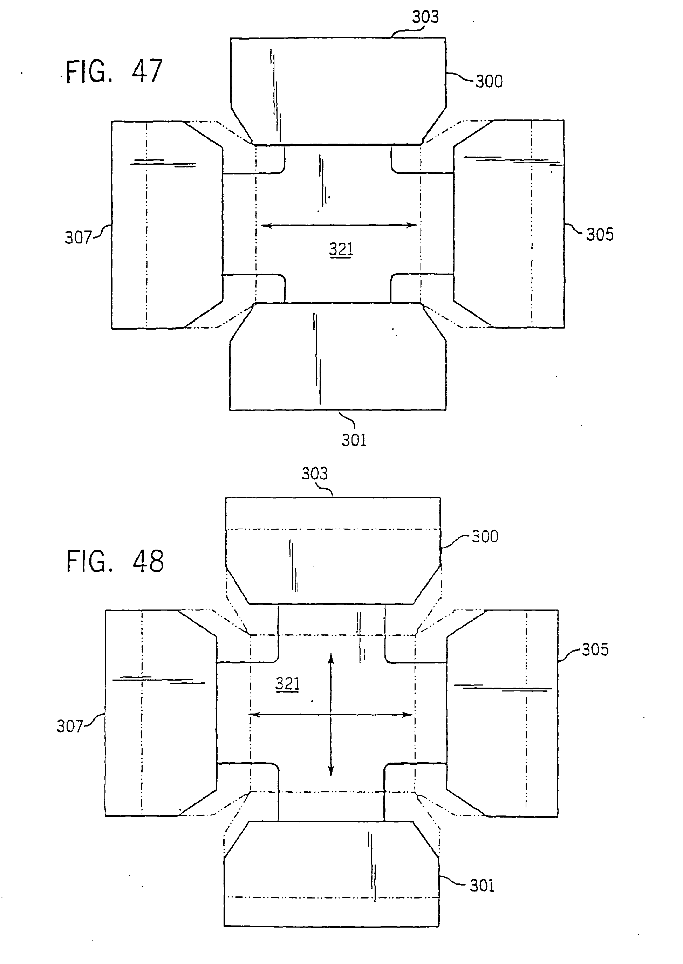

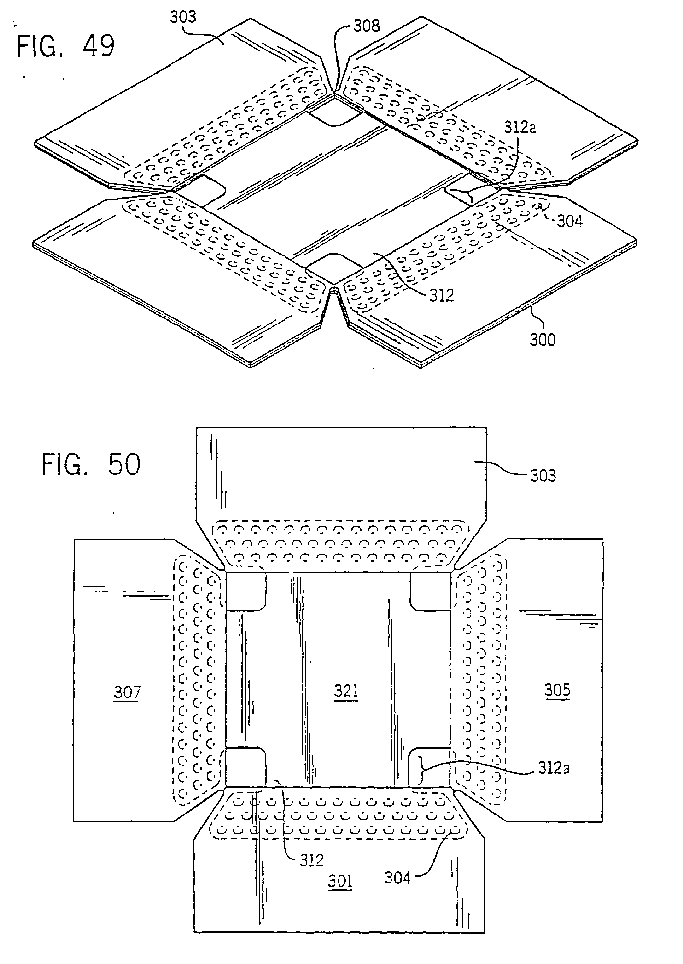

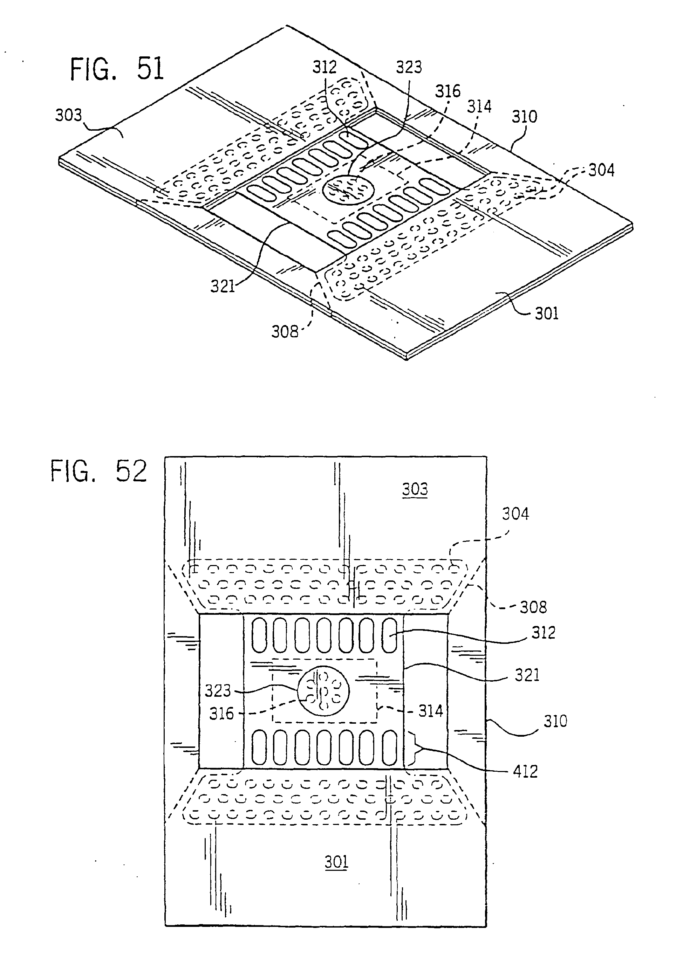

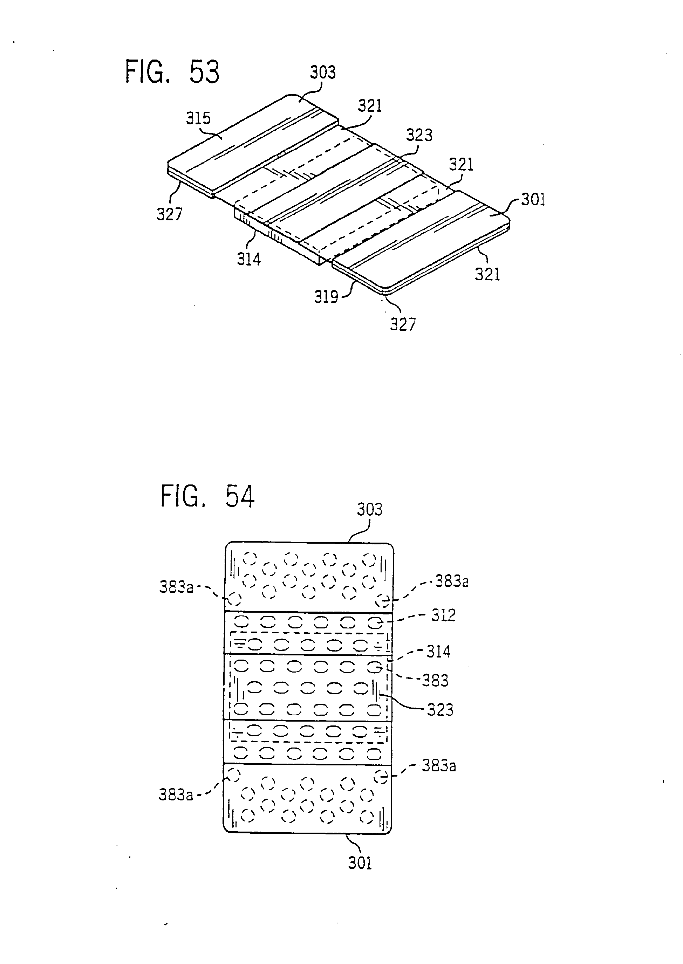

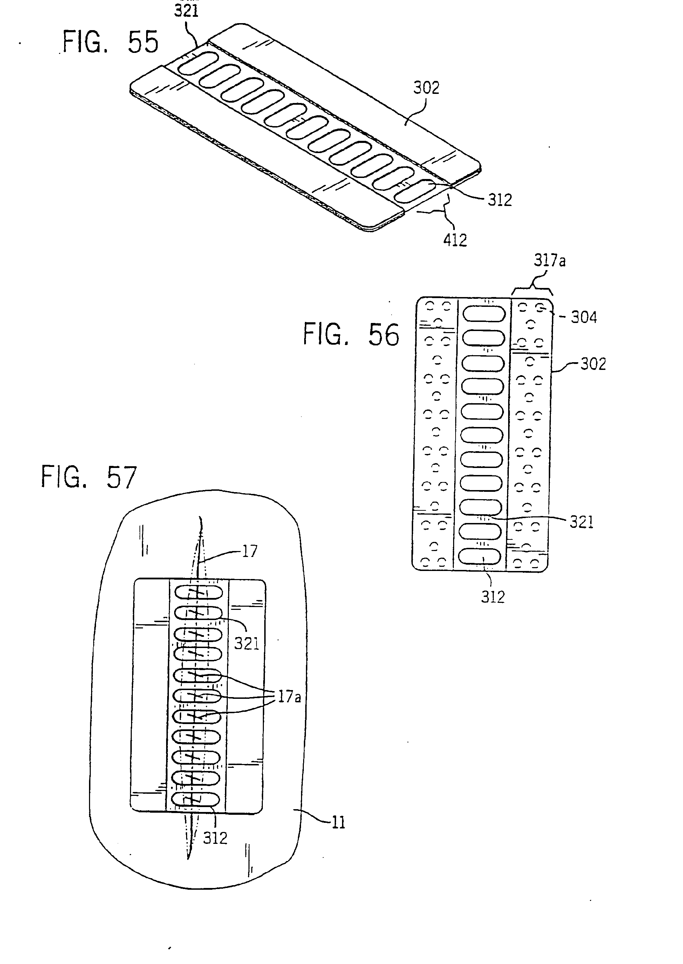

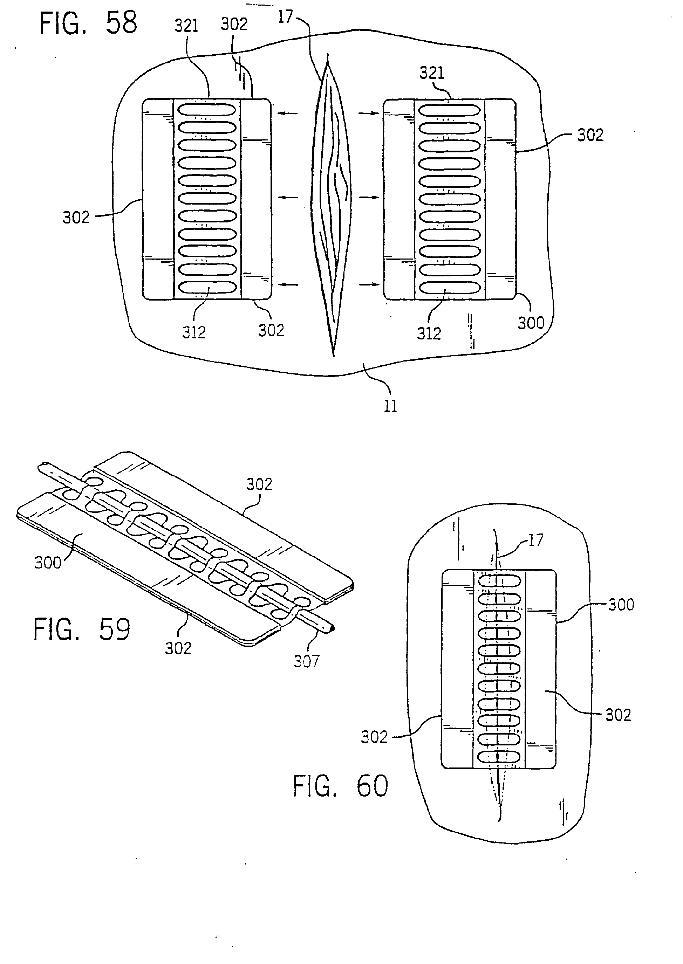

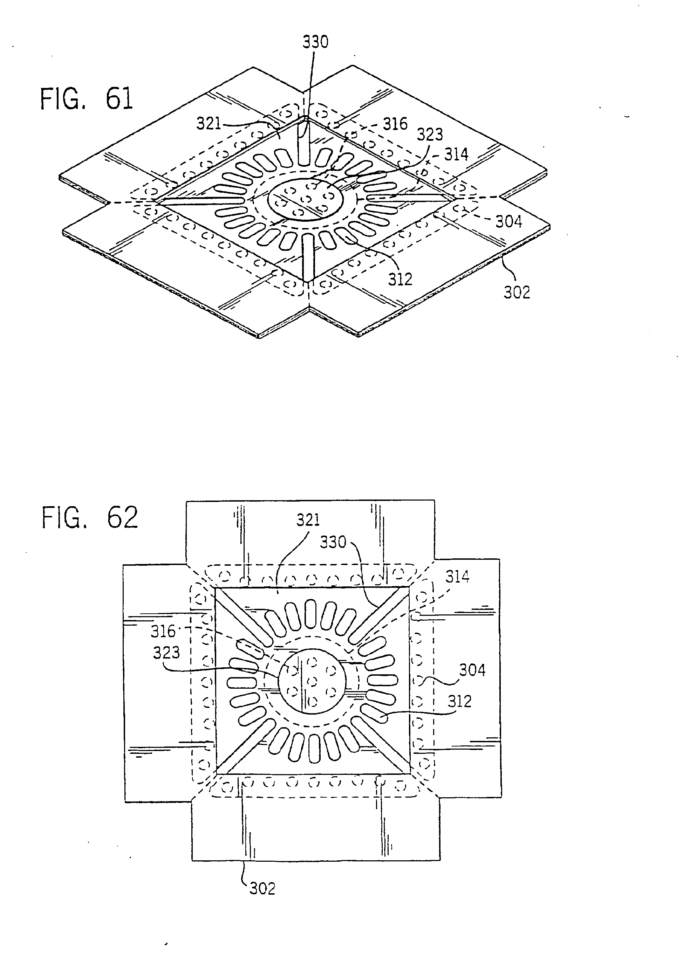

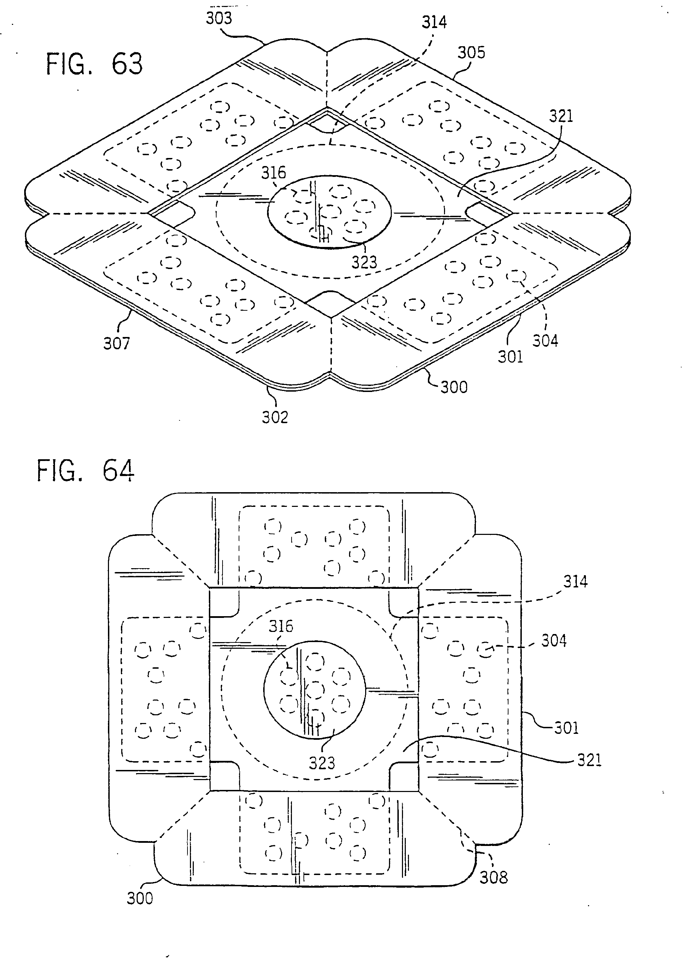

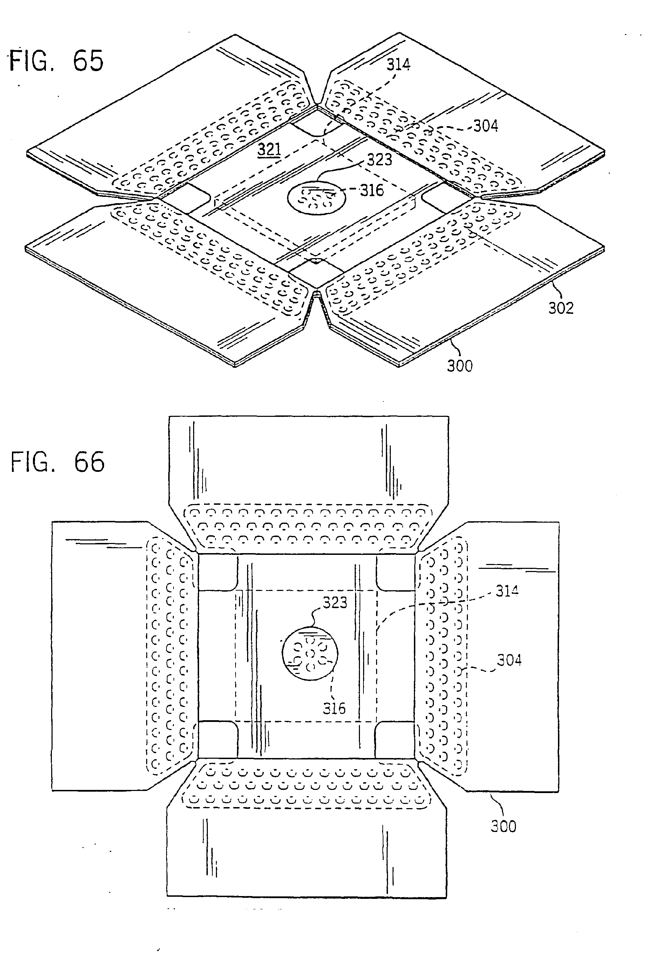

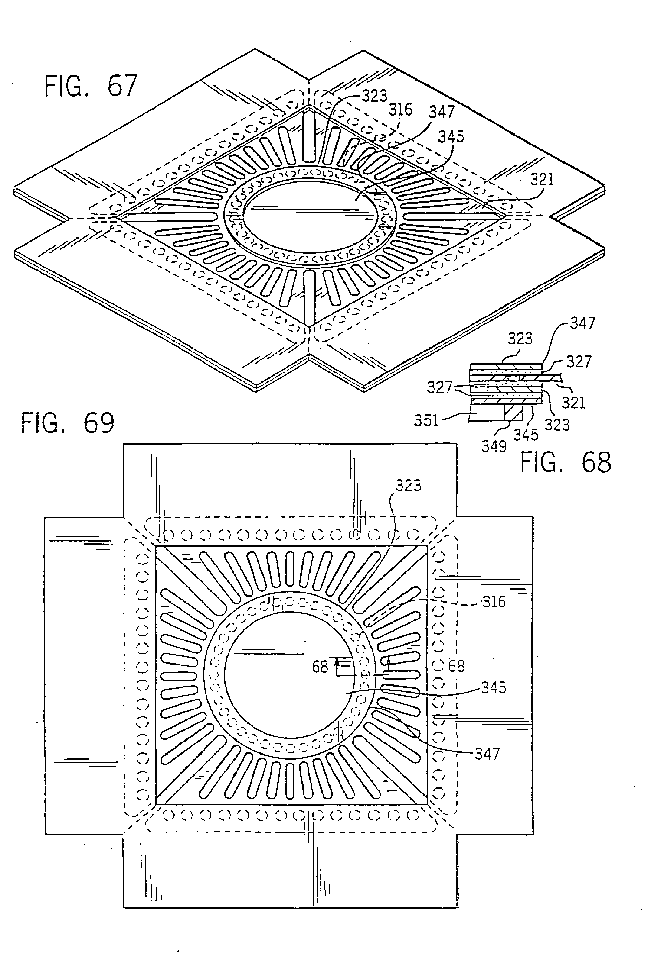

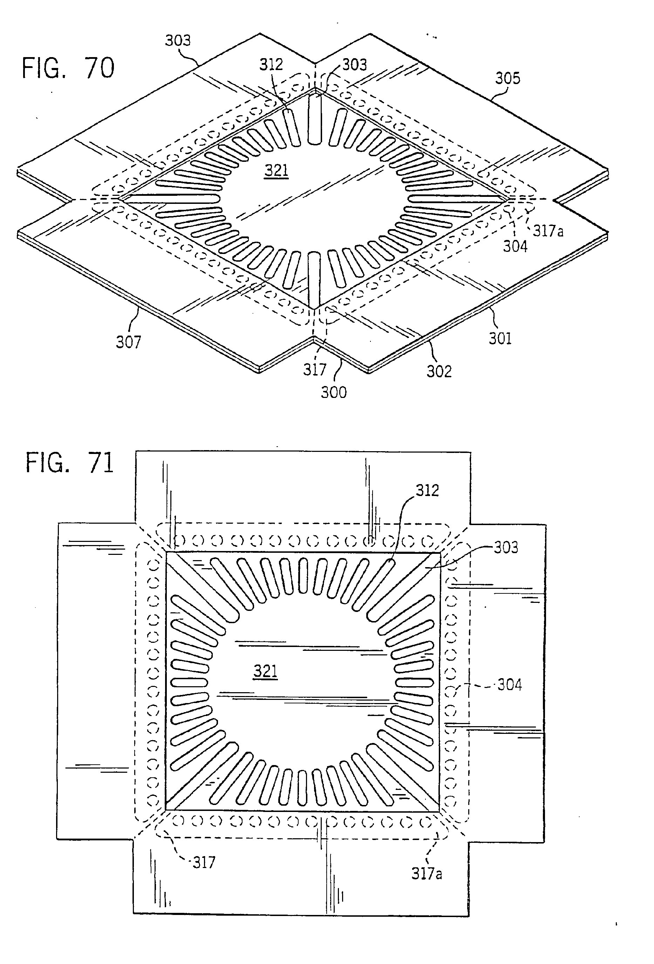

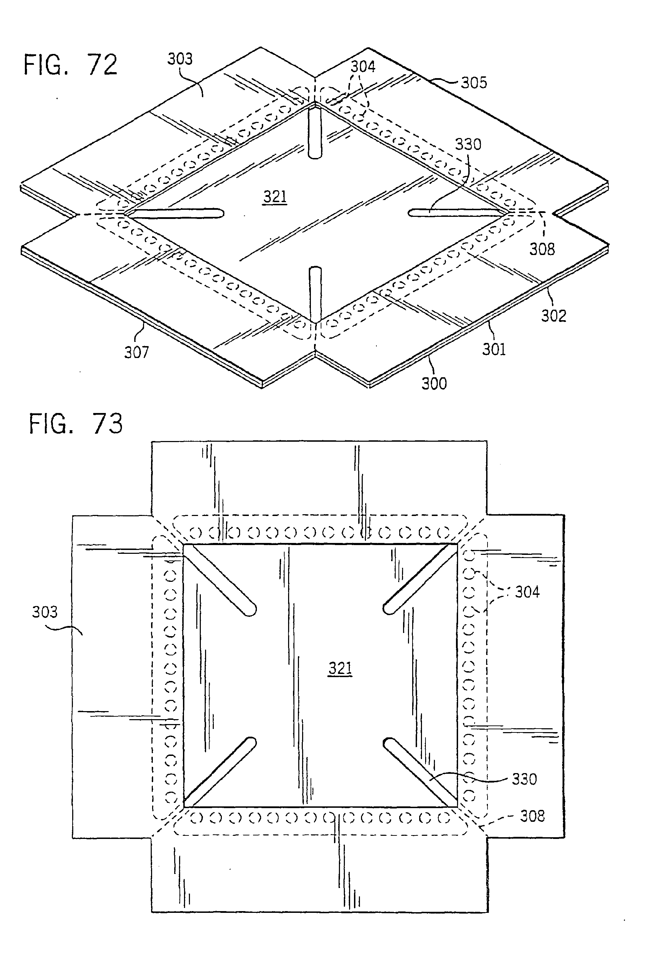

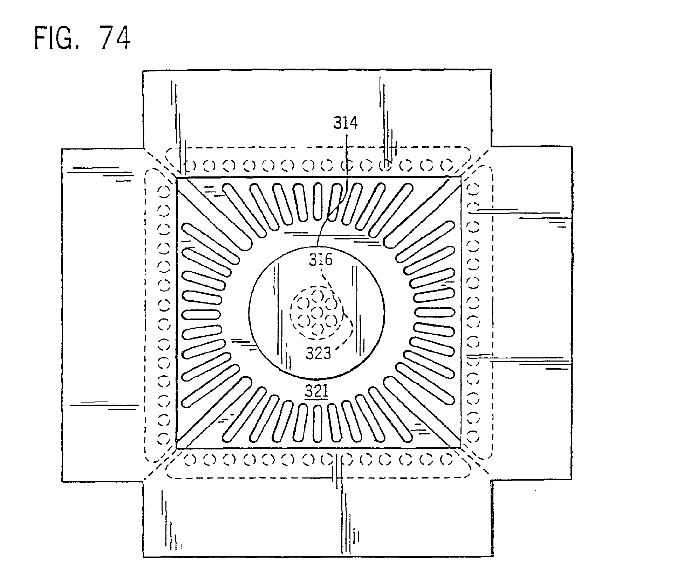

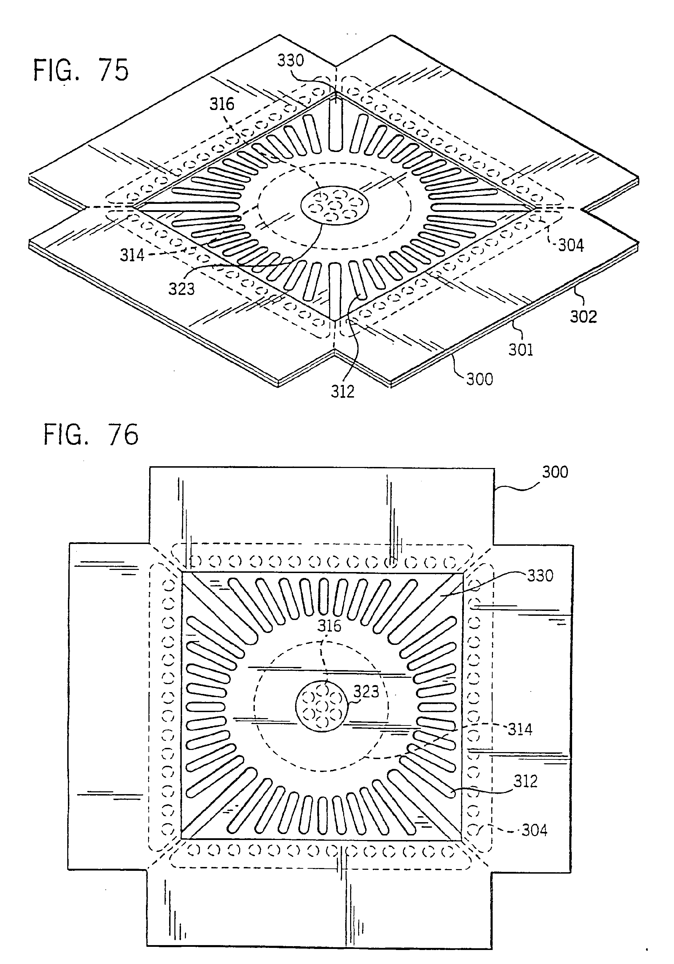

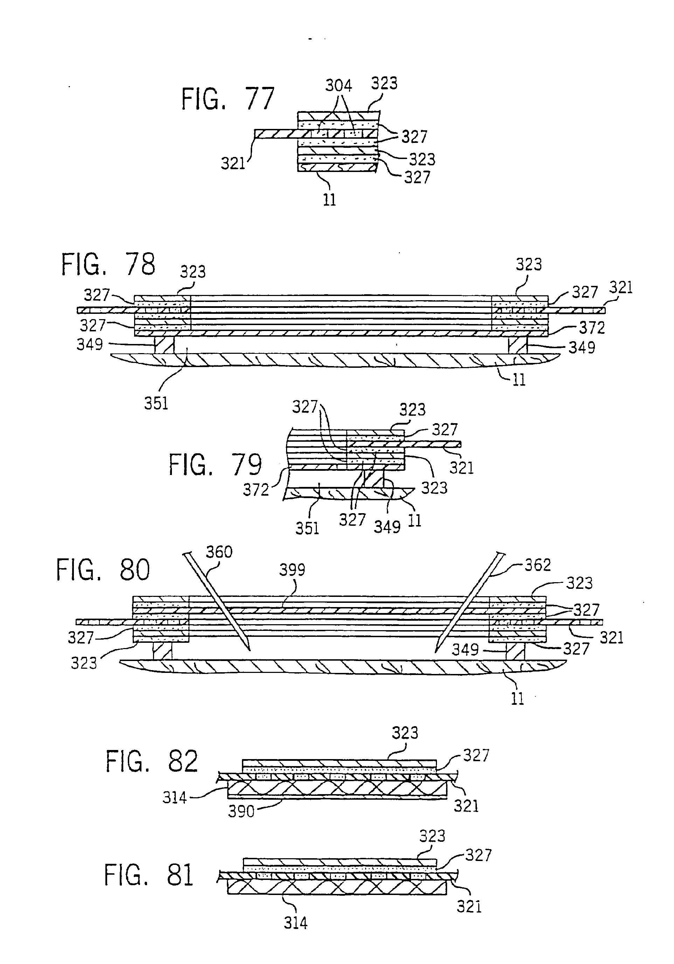

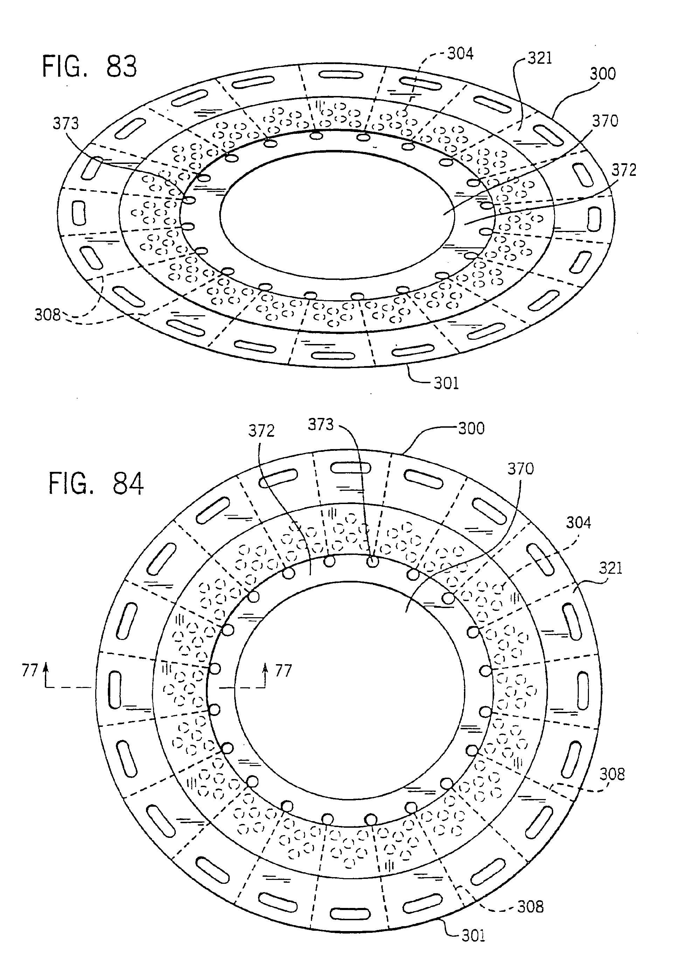

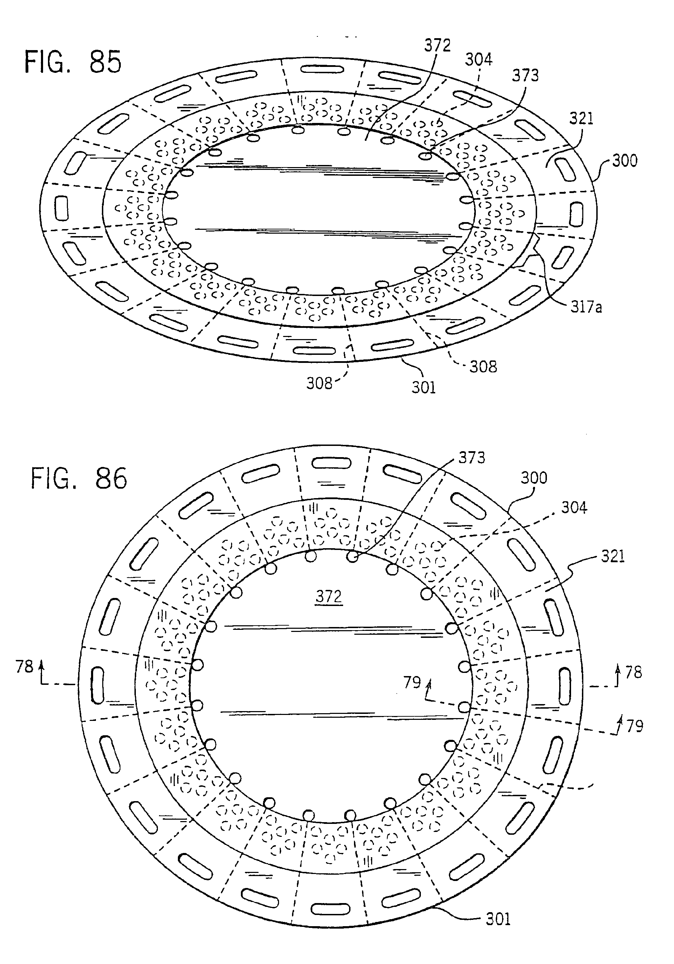

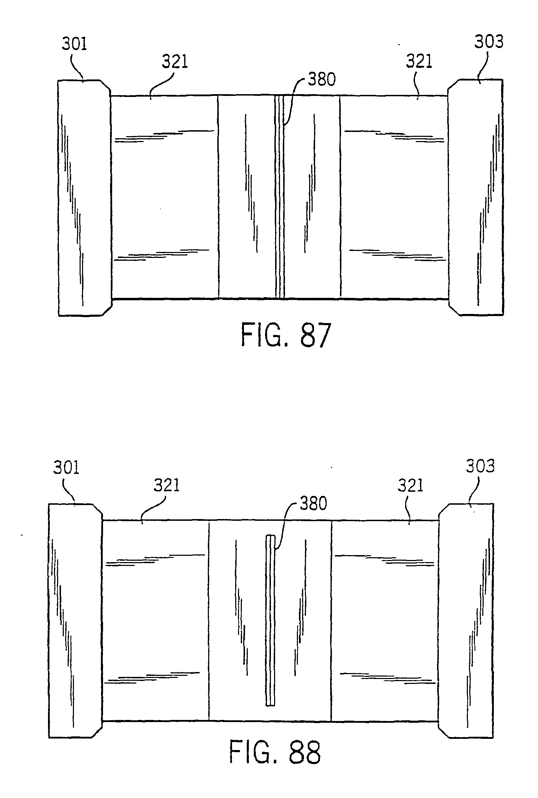

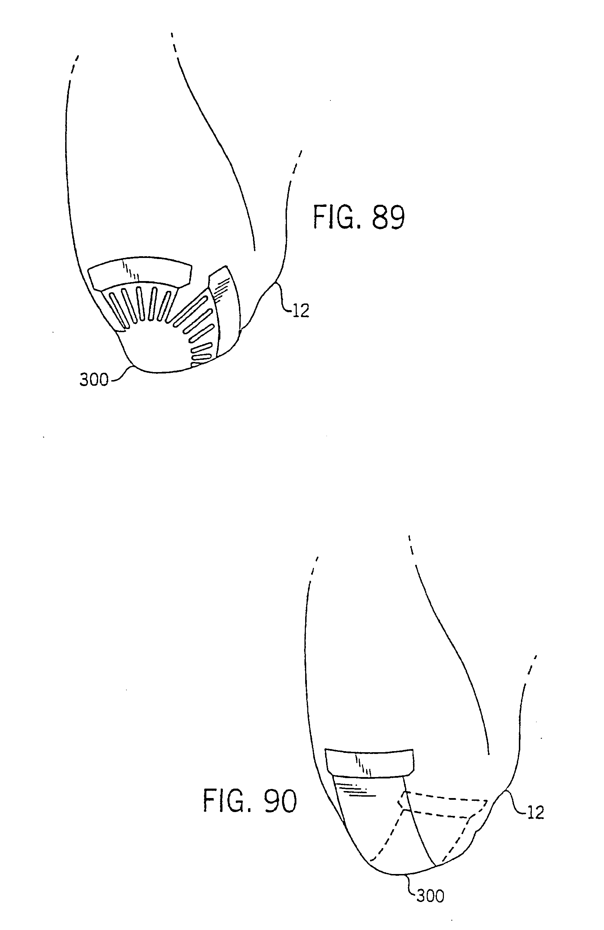

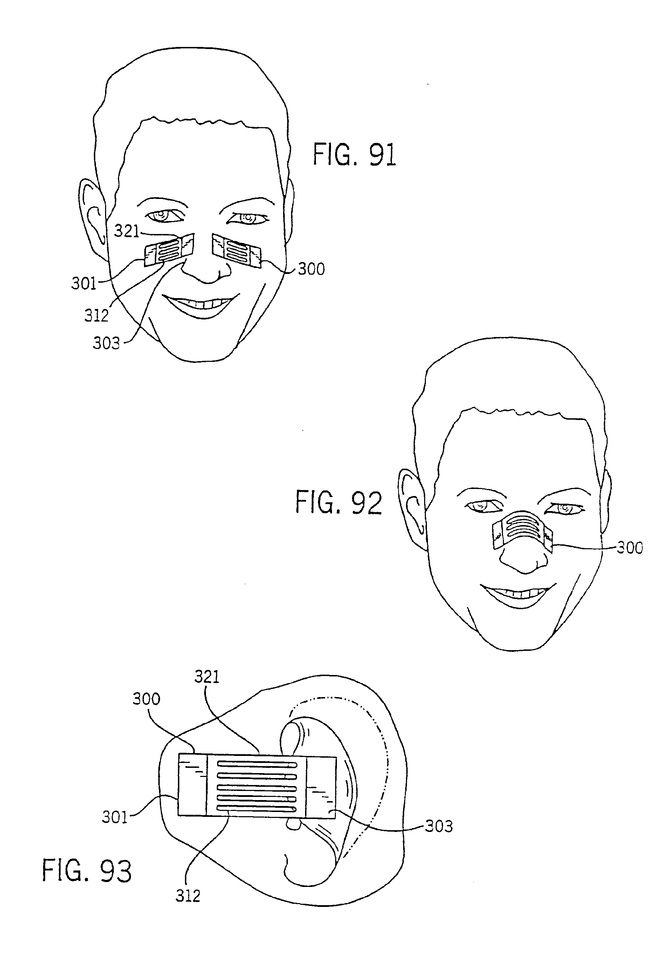

Referring now to Figures 28, 29 and 30; Figure 28 shows the nose 19 and the nasal passages 119 in cross sectional view. The nasal passages in Figure 28 being shown open but absent the use of any nasal dilator. In Figure 29 the same cross sectional view is shown but the nose 19 and in particular the nasal passages 119 are shown being in a state of relatively little airflow through the nasal passages 119. Figure 30 illustrates a cross sectional view using a nasal dilator of the present invention 10 wherein the nasal passages 119 of the nose 19 are held substantially open for airflow through the nasal passages 119.

Additionally, it should be noted that a medicinal material could be applied to the elastic member 110 over the portion of its surface which would be adjacent to the wound or incision 17 and thus aid in healing of the wound. Medicinal materials such as zinc chromate or calcium alginate or sodium alginate are possible such compounds.

Alternatively, the embodiment of Figure 21 could be used in a method wherein the pod 60 is applied to an epidermal area which is desired to be pulled or raised. This epidermal area could be an area immediately adjacent an incision or wound 17 or the side epidermis of a persons nose 19. The elastic member 110 being stretched and the anchor portion 120 being applied with its adhesive side to an epidermal area which is relatively stationary and the elastic material 110 contracting and thereby raising or pulling or lifting the skin to which the pod 60 has been attached to via its adhesive side.

Referring now to Figures 35 and 36 another alternative embodiment of the present invention may be observed. The dressing structure 300 is comprised of a multiple layer or laminated material 302 at its anchor section 301 and 303 and a latex rubber 321 at its center section 325. The laminated material includes a top surface 315 made of TYVEC brand material and a bottom surface 319 also made of the same material but coated with a hypo-allergenic acrylic adhesive 327 and covered with a silicone release liner. The anchor sections 301 and 303 have an adhesive bottom layer 311 for adhering to an epidermis 11. The laminated material 302 has a channel or slit 313 into which margins 317 of the latex rubber 321 are engaged. The margins 317 include openings 304 and the channel 313 includes the adhesive 327 which extends through the openings 304 from the bottom 319 to the top 315. This creates a series of adhesive openings 304 which act as plugs which extend through the openings 304 and couple the upper layer 315 to the lower layer 319 effectively holding the non-elastic TYVEC material together so that the latex material 321 is effectively locked into the channel 313 and cannot easily be removed by tension when stretched. Accordingly, margins 317 are secured to the anchor sections 301 and 303 at locking section 317a.

Still referring to Figure 35 and Figure 36 the center section 325 may be observed to include a TYVEC brand material stabilizing section 323 which is bonded to a gauze pad 314 via openings 316, in the latex 321 which contain adhesive 327. The adhesive 327 extending in a plug like manner from the pad 314 to the stabilizing section 323. This creates a bandage or dressing structure which is suspended by the latex 321 between the anchoring sections 301 and 303. Further, as illustrated by Figures 51 and 52 the shape of the TYVEC top layer 323 need not be rectangular but can be of any design, e.g., round. When this embodiment is applied over a wound or other predetermined area of the epidermis 11 the latex material 321 is stretched between the two anchoring sections 301 and 303 which causes the latex 321 to act much like a leaf spring and apply a positive pressure downward through the pad 314. Accordingly, the wound to which this device 300 is applied will have a positive pressure against it. It is well known in first aid that pressure applied to a wound will help reduce bleeding. The present invention thus provides an effective bandage which will also effectively limit bleeding from the wound. Further, the positive down pressure will effectively maintain contact of the pad 314 with the wound or other predetermined area despite movement of the surrounding epidermis 11.

Still referring to Figures 35 and 36 it should be noted that stability strips 310 are included to illustrate that it is presently believed that in commercial utilization of the present invention that it is believed to be desirable to provide material to keep the dressing structure 300 relatively rigid prior to use. The strips 310 are removed prior to use by tearing the material 302 along the perforations 308. The strips 310 are separated from the latex 321 by gap 318. Also, shown in Figure 36 is curve 320 which is believed to provide strain relief when the present dressing structure 300 is applied so that even pressure is exerted across the latex 321.

The openings 312, also shown in Figures 37 and 38, should also be noted. The openings 312 are located in a tension adjustment section 412 of the latex 321. Depending upon the number of openings 312 or whether they are present at all the tension applied to the latex section 321. Further, as the tension adjustment section 412 of the latex 321 is stretched to apply the dressing structure 300 the openings 312 will become distorted. The greater the stretching the greater the tension applied to the latex section 321. Consequently, a person applying the dressing structure disclosed herein may visually see the amount of tension applied to the latex section 321. This allows a person applying a dressing 300 or series of dressings 300 to apply the dressings 300 in a manner so that the pressure and exerted by the stretching of the latex 321 is kept relatively constant. Alternatively, it allows the user to apply dressings 300 which will apply a variety of pressures across the desired treatment area.

Referring to Figures 37 and 38 an alternative embodiment from that shown in Figures 35 and 36 may be seen wherein the pad 314 and inelastic material 323 are not incorporated so that only an elastic section 322 remains.

Referring to Figures 41 and 42, and Figures 45 and 46, another alternative embodiment to the present invention is illustrated. This embodiment is substantially the same structurally as the embodiments disclosed in Figures 35 and 36 with the exception that two additional anchors sections 305 and 307 have been added. Also, the stabilizing section 323 is round rather than rectangular in shape. The pad 314 is coupled to the stabilizing section as previously described. Figures 40 and 41 illustrate that tension adjustment sections 412 need not all apply the same level of tension or be stretched equally. Further, the anchor sections 301,303, 305, and 307 may be moved relative to each other while the center section 325 is maintained in position over the desired treatment area. Accordingly, when the present invention is applied over an area of the body that is subject to movement such as an elbow, knee, or hand the center section 325 will maintain its position over the wound or area to which it is desired to apply treatment.

Referring to Figures 39 and 40 another alternative embodiment may be observed. In this alternative the openings 312 have been eliminated to illustrate that they are optional and not necessary structures to practice the present invention.

Additionally, the stabilizing section disclosed in Figure 40 may be seen in Figure 82 to be composed of a top layer 323 of TYYEC brand material, a layer of adhesive 327, a layer of latex 321 having openings 304, and a pad 314 to which an ointment 390has been applied. The pad 314 being coupled to the material 323 via the adhesive 327 which extends through the openings 304 in the latex 321.

The stabilizing section disclosed in Figure 39 may be seen in Figure 81 to be composed of a top layer 323 of TYVEC brand material, a layer of adhesive 327, a layer of latex 321 having openings 304, and a pad 314. The pad 314 being coupled to the material 323 via the adhesive 327 which extends through the openings 304 in the latex 321.

Referring to Figures 43 and 44 another alternative embodiment of the present invention may be seen. In this embodiment four anchor sections are again shown coupled via respective locking sections 317a. In this embodiment just a latex material 321 extends between the anchor sections 301,303,305, and 307. A curvature 330 is provided in the latex material 321 to allow for uniform stretching of the material. Also, a perforation 308 is provided to connect the anchor sections 303,305,307 and 301 to each other prior to use of the dressing 300. The perforations are broken when it is desired to use this embodiment of the dressing 300.

Referring to Figures 47 and 48 it is again illustrated that the latex section 321 of the dressing 300 may be stretched or extended in a plurality of directions. This allows for versatility of use on a variety of surfaces.

Referring to Figures 49 and 50 another alternative embodiment of the present invention is disclosed showing that the openings 312 may be deleted from the tensioning section 312a if desired without detracting from the principles of the invention disclosed herein.

Referring to Figure 53 a very simple version of the present invention is illustrated. In this embodiment the dressing 300 is composed of a piece of latex 321 having two ends to which anchors 301 and 303 are respectively attached using an adhesive. The ends of the latex 321 are simply sandwiched between the layers 315 and 319. A piece of stiffening material 323 is glued across the mid-section of the latex 321 and pad 314 is glued to the underside of the latex 321 as illustrated. The bottom side of each respective anchor section 301 and 303 having an adhesive 327 applied thereto.

Referring to Figure 54 illustrates the embodiment of Figure 53 with the addition of a series of openings 383 being applied to the entire dressing 300. Depending upon the material through which the opening 383 is made the function of the opening will vary. Openings 312 in the latex 321 will act to vary the elasticity of the latex. Openings 383a will create stress points and help maintain the dressing 300 in a straight alignment between its anchors 301 and 303. Openings 383b will allow air access to the treatment area.

Referring now to Figures 55, 56, 57, 58, 59, and 60 another embodiment of the dressing 300, similar to the embodiment disclosed in Figures 37 and 38 is disclosed. In this embodiment the entire latex section 321 is essentially comprised of tensioning section 412 having openings 312. The anchors 301 and 303 function as previously described. The latex 321 in Figure 55 is held in place as described in Figure 53 while the latex 321 in Figure 56 is held in place as described in reference to Figures 35 and 36 by adhesive 327 extending through openings 304. Figures 57 - 60 illustrate that this embodiment may be placed over an incision 17 to act as a guided for applying stitches 17a, see Figure 57, or embodiments may be placed to either side of an incision 17 to hold the incision open, see Figure 58, or the openings 312 may be used to hold an intravenous tube 307 in place, see Figure 59, or the dressing 300 simply be used to hold an incision 17 closed without resorting to the application of stitches 17a, see Figure 60.

Referring to Figures 61 through 66 and Figures 70 through 76 a variety of alternative designs of the dressing 300 may be seen. All the dressings 300 disclosed operate on the same principles previously disclosed but they are shown to illustrate that shape of the latex 321 and the openings 312 may varied without departing from the invention described herein. Also, illustrated is the fact that the pad 314 and the material 323 may vary in size and shape. Further, the radius or arcuate section 330 may be varied in shape to provide for uniform distribution of tension across the latex 321.

Referring to Figures 67 through 69 another embodiment of the present invention may be seen wherein the latex 321 includes a ring section 347 of material 323. Coupled to the ring section 347 is the latex 321 and a clear urethane material 345 of the type commonly suitable for medical applications; alternative materials may be used such as any suitable breathable material depending upon the application desired. As illustrated by Figure 68 the ring section 347 is comprised of a layer of TYVEC brand material 323, a layer of adhesive 327, a layer of latex 321 having openings 316 which function in the same manner as openings 304, another layer of adhesive 327, another layer of TYVEC brand material 323, the clear material 345, and a colloid adhesive 349. This structure creates a stable space 351 over the desired area and the colloid 349 isolates the area and prevents stretching of the epidermis 11 under the space 351 so that the wound or other desired area is kept in an isolated environment which may be observed through the material 345. The colloid 349 and the material 345 isolating the wound from external sources of infection.

Referring to Figures 85 and 86 another alternative design of the present invention may be observed. In this embodiment the center section is a breathable membrane 372 of a type commonly used for dressing applications. Perforations 308 allow the dressing to be broken apart to form a plurality of anchor sections 301. Openings 373 are provided in the member 372 to prevent tearing of the membrane 372. A locking section 317a, previously described, is provided. Referring to Figures 78 and 79 the cross-sectional construction may be seen to include at top layer of material 323, a layer of adhesive 327, latex 321 including openings 304, adhesive 327, material 323, adhesive 327, the breathable membrane 372, and a colloid adhesive 349. The dressing 300 capable of covering a desired area of an epidermis 11 and substantially isolating that area from external contamination.

Referring now to Figures 83, 84, and 77 the same structures as shown in Figures 85 and 86, 78 and 79 are shown with the exception that the breathable membrane 372 has been eliminated so that there is only an opening 370. This dressing 300 is believed to have application where it is desired that the wounded or burnt area of the epidermis be exposed to air. Since the spring action of the latex 321 will press down on the epidermal area surround the wound or burn within the opening 370 this is believed to cause the wound or burn to well up and thus receive maximum exposure.

Referring now to Figure 80 another alternative embodiment similar to the structure disclosed in Figure 78 with the exception that the breathable membrane 372 has been replaced with an sealed membrane 399 such as a urethane commonly used to hold IV type fluids. Extending through this membrane 399 is an input port and an output port. This dressing 300 could be used to seal a wound from external contamination but allow the wound to be irrigated or medicine applied or tissue samples taken.

Referring now to Figures 87 and 88 another embodiment is illustrated showing a resealable closure 380. The closure or zipper 380 may bisect the dressing or extend only partially across the dressing 300. The closure 380 is provided to allow access to the wound or burn or other area without having to remove and reapply the bandage.

Referring now to Figures 89-93 various applications of the dressings 300 described herein may be seen to be illustrated in use on a human being.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A dressing mechanism, comprising
a first section (120; 301), a second section (110; 325) and a third section (120; 303), the first section (120; 301) being coupled to the second section (110; 325) and the second section (110; 325) being coupled to the third section (120; 303);
wherein the first section (301) and the third section (303) each have a first side, a predetermined portion of the first side having an adhesive coating,
the second section (110; 325) comprising an elastic material (321) including a plurality of openings (64; 312; 316),
**characterized in**
**that** the first section (120; 301) and the third section (120; 303) are each laminated materials comprising at least a first layer (319) and a second layer, and
**that** the first layer (319) includes the first side.

2. The dressing mechanism according to claim 1 wherein said second layer comprises an adhesive material.

3. The dressing mechanism according to claim 1 or 2 wherein the second section (110; 325) includes a first margin and a second margin, at least a portion of said first margin being located between the first side of the first layer and the second layer of said first section (120; 301); and wherein at least a portion of said second margin is located between the first side of the first layer and the second layer of said third section (120; 303).

4. The dressing mechanism according to claim 1 wherein the second section (110; 325) includes a first margin and a second margin, the first section (301) being integral to the second section (110; 325) at the first margin, the third section (120; 303) being integral to the second section (110; 325) at the second margin.

5. The dressing mechanism according to one of claims 1 to 4, wherein the openings (64; 312; 316) are of predetermined size.

6. The dressing mechanism according to one of claims 1 to 5, wherein the openings (64; 312; 316) are of predetermined shape.

7. The dressing mechanism according to claim 6 wherein the openings (64; 312; 316) of predetermined shape are spatially organized in a predetermined manner respective to each other.

8. The dressing mechanism according to one of claims 1 to 7 wherein the first layer (319) of the first section (301) and the first layer (319) of the third section (303) comprise an inelastic material.

9. The dressing mechanism according to one of claims 1 to 8 wherein the second layer of the first section (301) and the second layer of the third section (303) comprise an inelastic material.

10. The dressing mechanism of claim 8 or 9 wherein the inelastic material is polyester.

11. The dressing mechanism according to one of claims 1 to 10 wherein the first section (301) and the third section (303) each comprise a third intermediate layer.

12. The dressing mechanism according to claim 11 wherein the second section (110; 325) includes a first margin and a second margin, wherein the first section (301) includes a first channel (313) located between the first layer (319) and the second layer (315) of the first section (301) for receiving the first margin, and wherein the third section (303) includes a second channel (313) located between the first layer (319) and the second layer (315) of the third section (303) for receiving the second margin, the third intermediate layer comprising an adhesive material (327), the first margin and the second margin respectively including at least one opening (312), the first margin engaging the third intermediate layer in the first channel (313) and the adhesive material (327) extending through the opening (313) of the first margin, the second margin engaging the third intermediate layer in the second channel (313) and the adhesive material (327) extending through the opening of the second margin.
